Europäisches Patentamt

European Patent Office

Office européen des brevets

(19)

(11) EP 0 789 240 A1

(12) **EUROPEAN PATENT APPLICATION**

(43) Date of publication:
**13.08.1997   Bulletin 1997/33**

(51) Int Cl.6: **G01N 33/24**, G01N 33/18

(21) Application number: **97200403.0**

(22) Date of filing: **12.02.1997**

(84) Designated Contracting States:
**BE DE FR GB NL**

(30) Priority: **12.02.1996   NL 1002311**

(71) Applicant: **Heidemij Realisatie B.V.**
**5141 PA Waalwijk (NL)**

(72) Inventors:
• **Ten Brummeler, Erik**
  **5256 BE Heusden (NL)**
• **Bovendeur, Jan**
  **6871 TB Renkum (NL)**

(74) Representative: **Van Someren, Petronella F. H. M.**
**Arnold & Siedsma,**
**Advocaten en Octrooigemachtigden,**
**Sweelinckplein 1**
**2517 GK Den Haag (NL)**

(54)    **Method for predicting the result of a cleaning process**

(57)    The present invention relates to a method for predicting the end result of a biological cleaning process, comprising of taking a representative sample of the material for cleaning; characterizing the sample by determining a number of characteristic parameters; optionally separating the sample to size in order to obtain a fraction with coarse contaminants and a fraction with soil material; separating the soil material into a fraction containing the contaminants and a relatively clean fraction; if necessary conditioning the fraction containing the contaminants; incubating the conditioned fraction; measuring a number of cleaning parameters a number of times during the incubation in order to obtain measured values; and determining the quality of the soil after cleaning on the basis of the measured values obtained in the foregoing step.

## Description

The present invention relates to a method for predicting the end result of a biotechnological cleaning process of water, soil and organic residual flows.

Soil contamination and water pollution are serious environmental problems. In order to tackle these problems contaminated soil, optionally after excavation, and water can be cleaned by removing the contaminants. The soil can thereafter be spread again and the water discharged or re-used.

Organic residual flows, such as VFG waste, which may or may not be composted, (fermented) manure, sewage sludge (of compact consistency) and verge grass, may also still contain contaminants before they can be considered suitable for further use or re-use. These contaminants must of course first be removed.

For removal of contaminants from the various substrates different cleaning techniques can be applied. In addition to thermal and physical/mechanical methods, bio(techno)logical cleaning methods are being used increasingly often. In the Netherlands for instance over 200,000 tons of soil, mostly contaminated with mineral oil, is cleaned biotechnologically per annum.

In such biotechnological cleaning methods the contaminants are degraded microbiologically. The advantage of biotechnological cleaning is that the soil more or less retains its original structure, that the end products of the cleaning ($H_2O$ and $CO_2$) are harmless and that very little energy is required herein.

Biotechnological cleaning is based on the activity of micro-organisms already present in the soil. Although the contamination of the soil usually consists of oil and this is a very simple substrate for the micro-organisms already present, oil degradation in the soil does not however proceed spontaneously, or hardly so. Biotechnological cleaning therefore takes place, after excavation, under controlled conditions in an installation in which the ambient conditions such as temperature, moisture content and oxygen concentration can be geared precisely to the requirements of the desired micro-organisms. The result is that the degradation processes hereby either do take place or progress better.

The choice of the correct cleaning method and associated conditions for a determined type of material (soil or water) depends on a number of factors. The type of contamination is thus of importance and also the age of the contamination, the particle size distribution and the like. Whether cleaning of the contaminated material should be carried out at all depends on the result which can be obtained with cleaning. Soil which even after cleaning does not comply with legal requirements can better be stored than cleaned.

It is therefore important to be able to predict the result of the cleaning. An accurate cost estimate can thus be made and the quality of the end product can be predicted. Only when there is a well-substantiated expectation as to the final quality of the soil after cleaning can it be determined whether it is advisable to start the cleaning.

It is the object of the present invention to provide a method with which the final quality of the cleaned soil can be predicted and optionally a well-substantiated estimate can be given of the time the actual cleaning on large scale will take.

This is achieved by the invention with a method comprising the steps of:

a) taking a representative sample of the material for cleaning;
b) characterizing the sample by determining a number of characteristic parameters;
c) optionally separating the sample to size in order to obtain a fraction with coarse contaminants and a fraction with soil material;
d) separating the soil material into a fraction containing the contaminants and a relatively clean fraction;
e) if necessary conditioning the fraction containing the contaminants;
f) incubating the conditioned fraction;
g) measuring a number of cleaning parameters a number of times during the incubation in order to obtain measured values; and
h) determining the quality of the soil after cleaning on the basis of the measured values obtained in step g).

"Quality" is understood to mean inter alia the residual concentration of contaminants, but may also include the organic matter content, the "maturity" (stability) of the soil and the possible presence of heavy metals.

The sample can be a water or soil sample, but also a sample of organic material. The method according to the invention can in fact be used to predict the end result of a biotechnological cleaning of soil, groundwater, waste water and water channel beds, but also sewage sludge and fermented or non-fermented manure, verge grass or VFG waste.

The characteristic parameters determined before the sample is separated comprise for instance the particle size distribution, organic matter content and type and age of the contaminants. A separation schedule is formulated on the basis of this data. Depending on the values a choice can for instance be made as to whether or not to apply determined separation techniques.

Separation of the sample to size to obtain a fraction with coarse contaminants and a fraction with soil material is for instance carried out by means of sieving. Gravel, stones, branches and the like are hereby removed.

The separation into a fraction containing the contaminants and a relatively clean fraction often entails in practice a separation into a fine fraction and a coarse fraction. The coarse fraction comprises mainly sand, while the fine fraction consists substantially of fine sludge. It is known that most of the contaminants collect in the sludge fraction. It is for this reason that this fraction will usually be further used in the test.

Separation of the soil material into a fine fraction and a coarse fraction is carried out for instance by means of hydrocycloning.

If necessary, the sludge is subsequently conditioned. This entails additives being added and the pH and temperature optionally being adapted. The additives are for instance nutrients for the micro-organisms or means for bringing the pH to a determined value. The conditioning has the object of creating conditions wherein the micro-organisms function optimally. In addition, by varying the conditions it can be determined which conditions can best be used in the large-scale cleaning.

After the conditioning follows the incubation, during which the actual microbiological degradation takes place. The incubation can be performed aerobically or anaerobically subject to the contamination and the micro-organisms present.

During the incubation a number of parameters is again measured, which in this application are designated with the term "cleaning parameters". The cleaning parameters can be one or more of the following: $O_2$ demand, $CO_2$ production, $CH_4$ production, concentration of contaminants. $O_2$ demand and $CO_2$ production are measured when the cleaning is performed by aerobic micro-organisms. In the case of an anaerobic cleaning the production of biogas ($CH_4$) is measured. Measurement of the progress of the concentration of the contaminants can be performed in both types of cleaning.

The cleaning parameters give important information concerning the presence of an active bacteria population in the sample, the potential microbiological degradability and toxicity of the contamination, the achieving of a determined residual concentration and the optimum value of the conditions, such as pH, temperature and nutrients.

When the bacteria population is small or inactive little respiration or biogas production will take place. When the contamination is toxic the micro-organisms will die and little activity will consequently be observed. It has been found that the residual concentration of contaminants found on a small scale is representative of the residual concentration attainable on a large scale. On the basis of the cleaning parameters it can moreover be predicted how rapidly the cleaning process can be performed on a large scale.

The cleaning parameters are determined on the basis of experience. This experience teaches that the speed measured on laboratory scale is higher than the actual speed on a practical scale. The extent to which this speed on a practical scale is lower depends on the ambient factors (temperature, oxygen content), the soil properties, the type of contamination (for instance type of oil) and the age (i.e. the period between the occurrence of the contamination and the moment of cleaning). On the basis of experience in practice the speed which is determined in the laboratory can be converted using an empirical mathematical formula. The general form of the formula is:

$$T(t) = T_{\square} {}^*f_1 {}^*f_{os} {}^*f_{ellb} {}^*f_{ver} {}^*f_{temp} {}^*f_{O2},$$

in which

$T(t)$ = actual cleaning period (in days)
$T_{\square}$ = speed, determined with BAT method (in days) the dimensionless speed factors (number value always greater than 0 and less than or equal to 1):
$f_1$ = lutum content of the soil
$f_{os}$ = factor organic matter content of the soil
$f_{ellb}$ = correction factor sludge content of the soil
$f_{type}$ = type of oil contamination
$f_{temp}$ = temperature factor
$f_{O2}$ = aeration factor

Statistical analysis of the test results has shown that the end concentration of biotechnological soil cleaning cannot be predicted properly on the basis of the number value of a number of specific parameters, such as organic matter, sludge content and oil content. An empirical formula can be defined here in accordance with the prediction of the cleaning speed on a large scale. In view of the complex relation between the residual concentration and the soil properties, the accuracy of the empirical formula is sufficiently great only on the basis of a large number of data of the cleaned soils. The data is collected in a databank.

$$C(p) = (f_1 {}^*a_1 + f_{os} {}^*a_2 + f_{ellb} {}^*a_3 + f_{age} {}^*a_4 + f_{type} {}^*a_6 + C_o {}^*a_8),$$

in which

$a_{1-n}$ = empirically determined constant
$C(p)$ = [end concentration on a large scale]
$C_o$ = initial concentration of contamination
$f_{age}$ = age of contamination
$f_1$ = lutum content of the soil
$f_{os}$ = factor organic matter content of the soil
$f_{ellb}$ = correction factor sludge content of the soil
$f_{type}$ = type of oil contamination

The present invention will be further elucidated with reference to the following example, which is only given by way of illustration and is not intended to limit the invention.

**EXAMPLE**

The starting point is a representative soil sample of 5 kg. Using a sieve a fraction of > 1 mm is separated therefrom. In a hydrocyclone the sand is further removed to enable a good sample to be taken and to prevent wear of the installation.

The remaining sludge in the form of a slurry with a dry substance content of 1-5% is placed in reactors of 9 litres. The pH is adjusted to 7.0 with HCl or NaOH.

Nutrients are subsequently also added. The quantity is based on the amount of contamination, in this case oil, which is found in the sample. In 9 litres of slurry there is 0.0045 kg of oil. 1.35 g of biomass can be produced herefrom. The gross biomass formula is $C_5H_7O_2NP_{1/-30}S_{1/100}$. Per experiment 0.2 g N ($NO_3$-N: $NH_4^+$-N = 1:1 mol/mol), 0.02 g P and 0.02 g S (as $SO_4^{2-}$) is then added.

In order to be able to make a good overview of the oil degradation, part samples are taken of the starting sample, the fraction of > 1 mm, the sand (underflow hydrocyclone), washing water, sludge (overflow hydrocyclone) and sludge in the reactors several times during the duration of the test (1-2 weeks). Of these samples the dry substance content, organic matter content and the content of mineral oil are determined. The sludge coming out of the cyclone as overflow is likewise used as zero sample for the slurry reactors.

The incubation takes place at approximately 30°C. A rapid degradation is hereby obtained.

The microbiological activity is determined by means of measuring the oxygen demand. This is a relative measure for the presence of bacteria which can degrade oil and the presence of toxic substances. The activity is measured by closing down the reactor for a period of a few hours and measuring the decrease in the oxygen content. By plotting the decrease of oxygen against the time and calculating the gradient the oxygen demand per litre of slurry or kilogram of soil can be determined. It is known from the literature that normal active soil consumes about 0.2 mg of oxygen per kg per hour at 20°C and about 0.4 mg per kg per hour at 30°C. These are the minimum values which must be found for the oxygen demand.

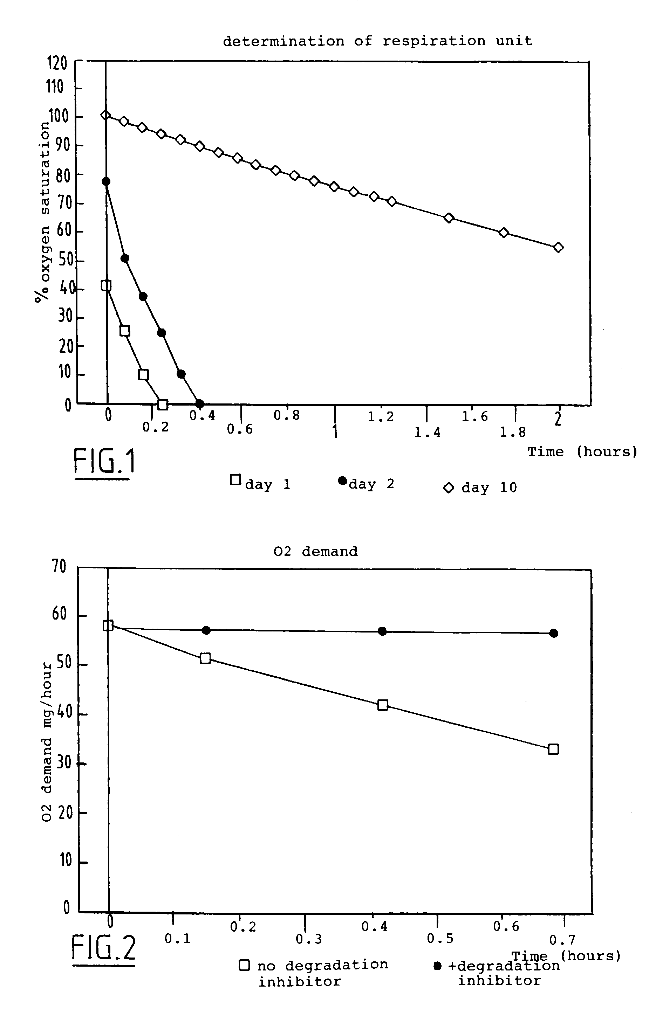

Figure 1 shows three curves representing the oxygen demand at different points in time. The slope of the curves corresponds with the maximum speed of oxygen demand. These show that the oxygen demand decreases sharply in time. This is of course related to a reduction in oil concentration in time.

It was checked whether the disappearance of the oil could be attributed to evaporation. For this purpose the oxygen demand and the oil degradation measured in samples (A), in which the micro-organisms were killed by means of sodium azide, were compared with ordinary samples (B) in which the micro-organisms were normally active. The result is shown in figure 2. The absence of any oxygen demand by samples (A) demonstrates that there were indeed no longer any micro-organisms present therein. Nor did these samples show any reduction in oil concentration.

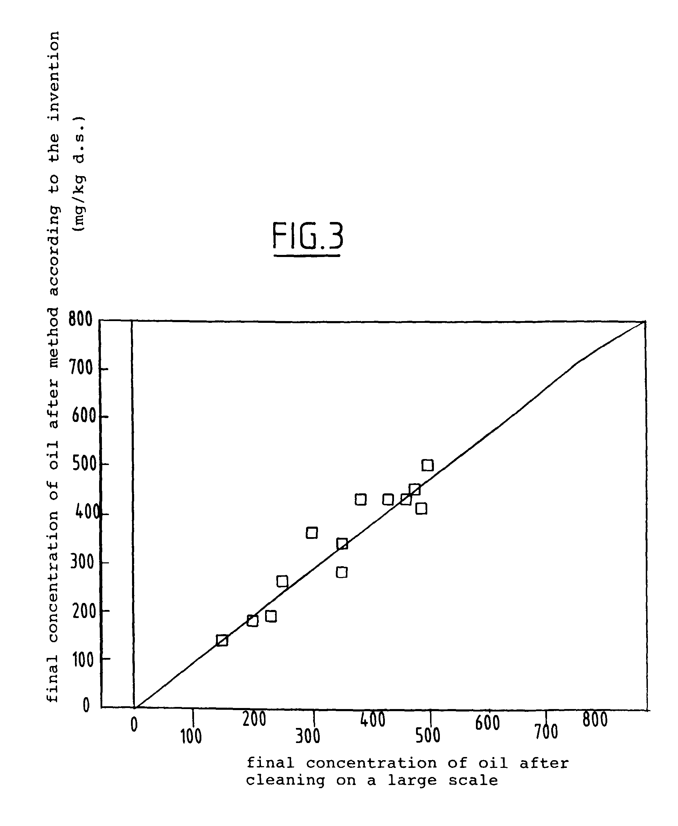

Figure 3 shows the correlation between the cleaning result after performing the method according to the invention and after cleaning on a large scale. This shows the predictive value of the method according to the invention.

**Claims**

1. Method for predicting the end result of a biological cleaning process, comprising the steps of:

   a) taking a representative sample of the material for cleaning;
   b) characterizing the sample by determining a number of characteristic parameters;
   c) optionally separating the sample to size in order to obtain a fraction with coarse contaminants and a fraction with soil material;
   d) separating the soil material into a fraction containing the contaminants and a relatively clean fraction;
   e) if necessary conditioning the fraction containing the contaminants;
   f) incubating the conditioned fraction;
   g) measuring a number of cleaning parameters a number of times during the incubation in order to obtain measured values; and
   h) determining the quality of the soil after cleaning on the basis of the measured values obtained in step g).

2. Method as claimed in claim 1, **characterized in that** the fraction containing the contaminants is the fine sludge fraction and the relatively clean fraction is the sand fraction.

3. Method as claimed in claim 1 or 2, **characterized in that** for further processing the fraction containing the contaminants takes the form of a slurry with a dry substance content of between 0.1 and 20%, preferably between 0.5 and 10% and more preferably between 1 and 5%.

4. Method as claimed in claim 1, 2 or 3, **characterized in that** the cleaning parameters measured during incubation consist of one or more of the following: $O_2$ demand, $CO_2$ production, $CH_4$ production, concentration of contaminants.

5. Method as claimed in any of the claims 1-4, **characterized in that** the conditioning of the fraction containing the contaminants consists of adding nutrients and optionally adjusting the pH and/or temperature to a desired value.

6. Method as claimed in any of the claims 1-5, **characterized in that** the characteristic parameters are chosen from particle size distribution, organic matter content, type of contamination, age of the contamination.

7. Method as claimed in any of the claims 1-6, **characterized in that** the material for cleaning is soil, groundwater, water channel beds, waste water, sewage sludge, fermented or non-fermented manure, verge grass, fermented or non-fermented VFG waste or a combination thereof.

8. Method as claimed in any of the claims 1-7, **characterized in that** separation of the sample to size to obtain a fraction with coarse contaminants and a fraction with soil material is carried out by means of sieving.

9. Method as claimed in any of the claims 1-8, **characterized in that** separation into a fraction containing the contaminants and a relatively clean fraction is carried out by means of hydrocycloning.

10. Method as claimed in any of the claims 1-9, **characterized in that** the prediction of the end result of the cleaning on large scale is based on the correlation between a number of cleanings on small and large scale carried out in parallel with each other.

determination of respiration unit

FIG.1

□ day 1    ● day 2    ◇ day 10

O2 demand

FIG.2

□ no degradation
  inhibitor

● +degradation
  inhibitor

## FIG.3

**European Patent Office**

## EUROPEAN SEARCH REPORT

Application Number

EP 97 20 0403

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | DE 43 33 490 A (DMT-GESELLSCHAFT FÜR FORSCHUNG UND PRÜFUNG MBH) 13 April 1995 * the whole document * --- | 1-10 | G01N33/24 G01N33/18 |
| Y | TRANSACTIONS OF THE INSTITUTE OF MEASUREMENT AND CONTROL, vol. 6, no. 3, July 1984, DORKING GB, pages 117-131, XP002016808 M.B.BECK: "MODELLING AND CONTROL STUDIES OF THE ACTIVATED SLUDGE PROCESS AT NORWICH SEWAGE WORKS" * the whole document * --- | 1-10 | |
| Y | US 5 266 494 A (LAHODA ET AL.) 30 November 1993 * the whole document * --- | 9,10 | |
| A | EP 0 531 955 A (FORSCHUNGSZENTRUM JÜLICH GMBH) 17 March 1993 * column 5, line 41 - column 10, line 20; figures 1-7 * --- | 1,4,5,7 | |
| A | FR 2 717 580 A (ALECTRICITA DE FRANCE) 22 September 1995 * the whole document * --- | 1,4,5,7 | **TECHNICAL FIELDS SEARCHED** (Int.Cl.6) G01N |
| A | DE 41 29 363 A (BERGWERKSVERBAND GMBH) 11 March 1993 * column 2, line 24 - column 5, line 52; figures 1-5 * ----- | 1,4,5,7 | |

The present search report has been drawn up for all claims

| Place of search | Date of completion of the search | Examiner |
|---|---|---|
| THE HAGUE | 28 April 1997 | Bosma, R |